# EUROPEAN PATENT APPLICATION

(11) **EP 1 464 678 A1**
(43) Date of publication of application: **06.10.2004**
(21) Application number: 04008244.8
(22) Date of filing: 05.04.2004
(51) Int. Cl.: C09B 57/00, G02B 5/00, G11B 7/24, C07D 209/12

(54) **Cyanine compound, optical filter, and optical recording material**

(30) Priority: 04.04.2003 JP 2003101725; 12.02.2004 JP 2004035683
(71) Applicant: Asahi Denka Co., Ltd., Tokyo (JP)
(72) Inventor: Shimizu, Masaaki, Arakawa-ku Tokyo (JP); Shigeno, Koishi, Arakawa-ku Tokyo (JP); Yano, Toru, Arakawa-ku Tokyo (JP)
(74) Representative: Forstmeyer, Dietmar, Dr. rer. nat., Dipl.-Chem.

(57) **Abstract**

A cyanine compound of formula (I), an optical filter containing the cyanine compound, and an optical recording material containing the cyanine compound which is used to form an optical recording layer of an optical recording medium: 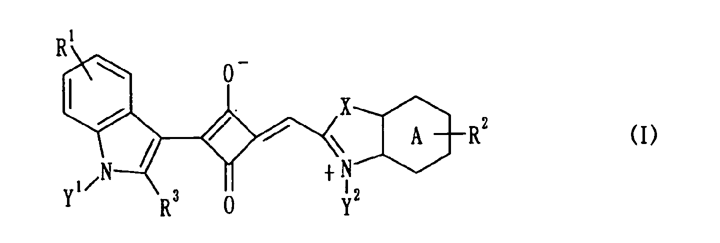 wherein ring A represents benzene or naphthalene; R¹ and R² each represent hydrogen, halogen, nitro, cyano, C1-C8 alkyl, C1-C8 alkoxy or C6-C30 aryl; R³ represents hydrogen, C1-C8 alkyl or C6-C30 aryl; X represents oxygen, sulfur, selenium, -CR⁴R⁵-, - NH- or -NY'-; Y¹ and Y² each represent hydrogen or C1-C30 organic group; R⁴ and R⁵ each represent C1-C4 alkyl or benzyl, or R⁴ and R⁵ are taken together to form C3-C6 cycloalkane-1,1-diyl; and Y' represents C1-C30 organic group.

## Description

### FIELD OF THE INVENTION

This invention relates to a novel cyanine compound, an optical filter, and an optical recording material. The cyanine compound of the present invention is useful as an optical element, particularly as a light absorber in an optical filter of an image display device or an optical recording agent used in a laser optical recording material.

### BACKGROND OF THE INVENTION

Compounds having a high absorption in a wavelength range of 500 to 700 nm, especially those having an absorption peak wavelength (λₘₐₓ) between 550 nm and 620 nm, are used as an optical element in an optical recording layer of optical recording media including DVD-Rs and in an optical filter of image displays including liquid crystal displays (LCDs), plasma display panels (PDPs), electroluminescent displays (ELDs), cathode ray tube displays (CRTs), fluorescent display tubes, and field emission displays (FEDs).

Cyanine compounds having a squarylium structure have been studied as a promising optical element for these applications. For example, JP-A-2002-228829 (compound Nos. 1 to 16), JP-A-2002-294094 (chemical formulae 1 to 26), and JP-A-5-339233 (Table 1) disclose cyanine compounds having an indole ring and a squarylium structure. However, these known compounds have poor resistance to light and/or heat and cannot be seen as satisfactory in duration of functions as an optical element.

### SUMMARY OF THE INVENTION

An object of the present invention is to provide a compound excellent in resistance to light and heat and suited as an optical element for use in an optical filter of image display devices or in a laser optical recording material.

As a result of extensive investigations, the present inventors have found a specific cyanine compound having a squarylium structure excellent in resistance to light and heat and reached the present invention.

Based on the above finding, the present invention provides a cyanine compound represented by formula (I) shown below (hereinafter referred to as a cyanine compound (I)), an optical filter containing the cyanine compound (I), and an optical recording material for use in an optical recording medium having a substrate and an optical recording layer formed on the substrate, the optical recording material containing the cyanine compound (I) and being used in the optical recording layer. 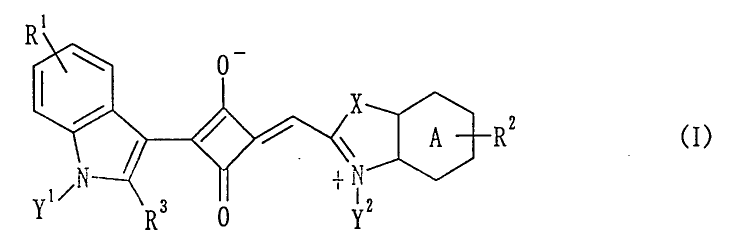 wherein ring A represents a benzene ring or a naphthalene ring; R¹ and R² each independently represent a hydrogen atom, a halogen atom, a nitro group, a cyano group, an alkyl group having 1 to 8 carbon atoms, an alkoxy group having 1 to 8 carbon atoms or an aryl-containing group having 6 to 30 carbon atoms; R³ represents a hydrogen atom, an alkyl group having 1 to 8 carbon atoms or an aryl-containing group having 6 to 30 carbon atoms; X represents an oxygen atom, a sulfur atom, a selenium atom, -CR⁴R⁵-, -NH- or -NY¹-; Y¹ and Y² each independently represent a hydrogen atom or an organic group having 1 to 30 carbon atoms; R⁴ and R⁵ each independently represent an alkyl group having 1 to 4 carbon atoms or a benzyl group, or R⁴ and R⁵ are taken together to form a cycloalkane-1,1-diyl group having 3 to 6 carbon atoms; and Y' represents an organic group having 1 to 30 carbon atoms.

In formula (I) representing the cyanine compound (I) of the present invention, the halogen atom represented by R¹ and R² includes fluorine, chlorine, bromine, and iodine. The alkyl group having 1 to 8 carbon atoms include methyl, ethyl, propyl, isopropyl, butyl, sec-butyl, tert-butyl, isobutyl, amyl, isoamyl, tert-amyl, hexyl, cyclohexyl, heptyl, isoheptyl, tert-heptyl, n-octyl, isooctyl, tert-octyl, and 2-ethylhexyl. The alkoxy group having 1 to 8 carbon atoms include methoxy, ethoxy, isopropoxy, propoxy, butoxy, pentyloxy, isopentyloxy, hexyloxy, heptyloxy, octyloxy, and 2-ethylhexyloxy. The aryl-containing group containing 6 to 30 carbon atoms include phenyl, naphthyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-t-butylphenyl, 4-hexylphenyl, 4-cyclohexylphenyl, 4-octylphenyl, 4-(2-ethylhexyl)phenyl, 4-stearylphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4-di-t-butylphenyl, 2,5-di-t-butylphenyl, 2,6-di-t-butylphenyl, 2,4-di-t-pentylphenyl, 2,5-di-t-amylphenyl, 2,5-di-t-octylphenyl, 2,4-dicumylphenyl, cyclohexylphenyl, biphenyl, 2,4,5-trimethylphenyl, benzyl, phenethyl, 2-phenylpropan-2-yl, diphenylmethyl, triphenylmethyl, styryl, and cinnamyl. The alkyl group having 1 to 8 carbon atoms and the aryl-containing group having 6 to 30 carbon atoms as represented by R³ include those enumerated above with respect to R¹.

The alkyl group having 1 to 4 carbon atoms as R⁴ and R⁵ in -CR⁴R⁵- representing X includes methyl, ethyl, propyl, isopropyl, butyl, see-butyl, t-butyl, and isobutyl. The cycloalkane-1,1-diyl group having 3 to 6 carbon atoms that is formed by R⁴ and R⁵ connected together includes cyclopropane-1,1-diyl, cyclobutane-1,1-diyl, 2,4-dimethylcyclobutane-1,1-diyl, 3-dimethylcyclobutane-1,1-diyl, cyclopentane-1,1-diyl, and cyclohexane-1,1-diyl. The organic group having 1 to 30 carbon atoms as Y' in X includes an alkyl group, e.g., methyl, ethyl, propyl, isopropyl, butyl, see-butyl, t-butyl, isobutyl, amyl, isoamyl t-amyl, hexyl, cyclohexyl, cyclohexylmethyl, 2-cyclohexylethyl, heptyl, isoheptyl, t-heptyl, n-octyl, isooctyl, t-octyl, 2-ethylhexyl, nonyl, isononyl, decyl, dodecyl, tridecyl, tetradecyl, pentadecyl, hexadeyl, heptadecyl or octadecyl; an alkenyl group, e.g., vinyl, 1-methylethenyl, 2-methylethenyl, propenyl, butenyl, isobutenyl, pentenyl, hexenyl, heptenyl, octenyl, decenyl, pentadecenyl or 1-phenylpropen-3-yl; an alkyl-substituted or unsubstituted aryl group, e.g., phenyl, naphthyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-t-butylphenyl, 4-hexylphenyl, 4-cyclohexylphenyl, 4-octylphenyl, 4-(2-ethylhexyl)phenyl, 4-stearylphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4-di-t-butylphenyl or cyclohexylphenyl; and an arylalkyl group, e.g., benzyl, phenethyl, 2-phenylpropan-2-yl, diphenylmethyl, triphenylmethyl, styryl or cinnamyl. The organic group as Y' further includes the above-recited organic groups which contain an ether linkage or a thioether linkage, such as 2-methoxyethyl, 3-methoxypropyl, 4-methoxybutyl, 2-butoxyethyl, methoxyethoxyethyl, methoxyethoxyethoxyethyl, 3-methoxybutyl, 2-phenoxyethyl, 3-phenoxypropyl, 2-methylthioethyl, and 2-phenylthioethyl. The organic groups recited above as Y' may be substituted with an alkoxy group, an alkenyl group, a nitro group, a cyano group, a halogen atom, etc.

The organic group having 1 to 30 carbon atoms as Y¹ or Y² includes the same groups as enumerated as Y'.

Methods for introducing Y', Y¹, and Y² into the skeleton of formula (I) are not particularly restricted. For example, Y¹ can be introduced by allowing the NH group of a 3H-indole derivative to react with an organic halide compound represented, e.g., by Hal-Y¹ (wherein Hal represents fluorine, chlorine, bromine or iodine). As the numbers of carbon atoms in Y', Y¹ and Y² increase, the compound of formula (I) increases in molecular weight, which can result in reduction of molecular extinction coefficient. Therefore, the carbon atom number of Y', Y¹ or Y² is preferably 20 or smaller, still preferably 10 or smaller.

Of the cyanine compounds (I), those in which X is -CR⁴R⁵-, i.e., compounds represented by formula (II) shown below are preferred for their excellent light resistance. 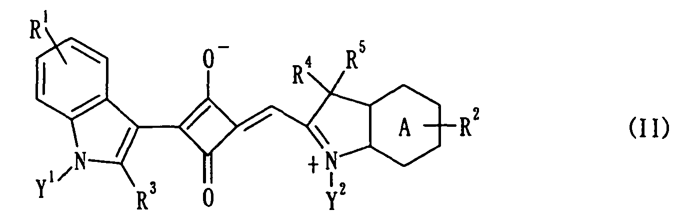 wherein ring A, R¹, R², R³, R⁴, R⁵, Y¹, and Y² are as defined above.

Specific examples of the cyanine compounds (I) of the present invention are shown below.

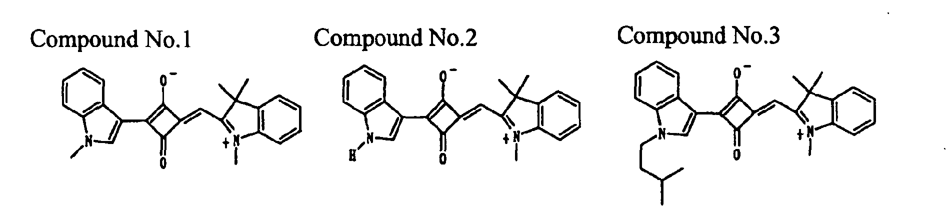

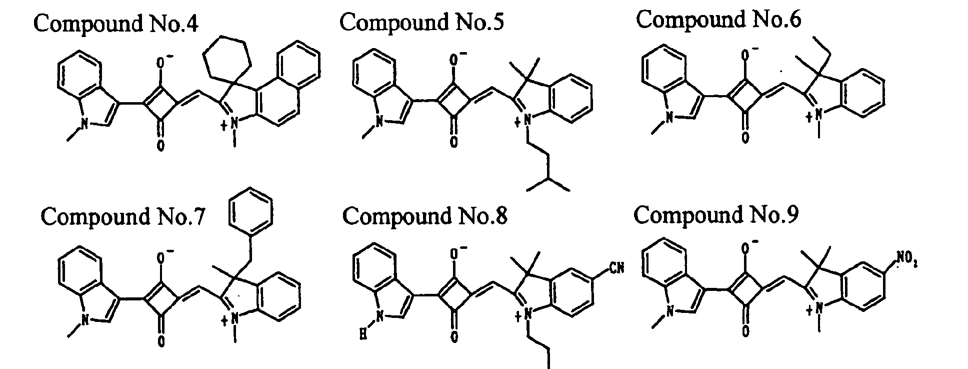

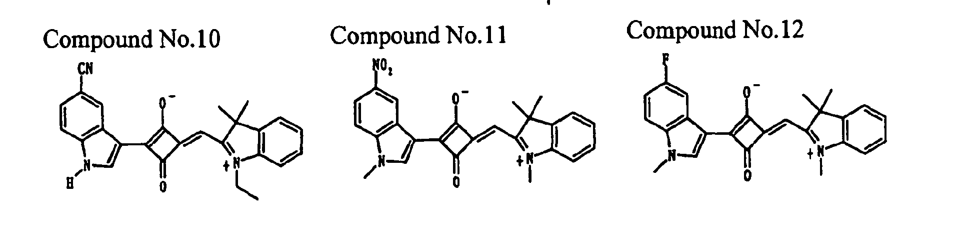

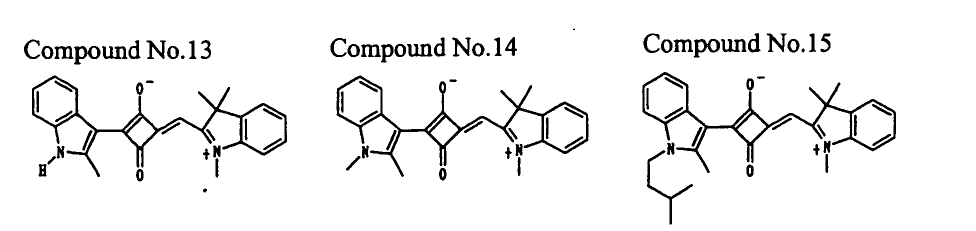

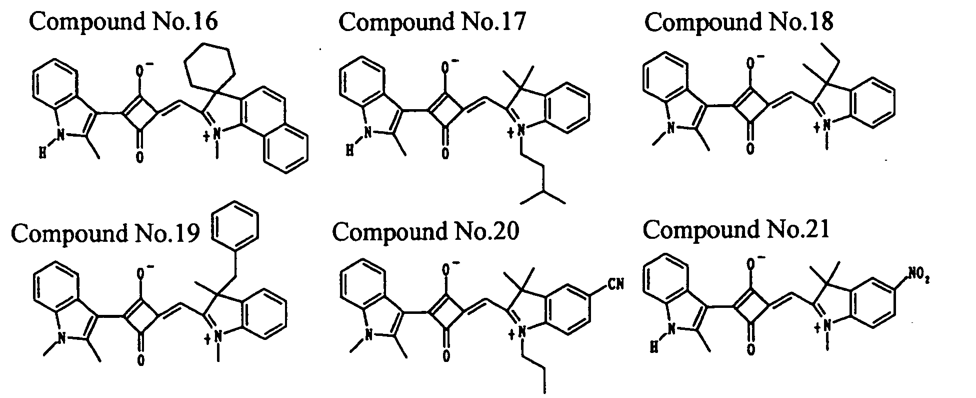

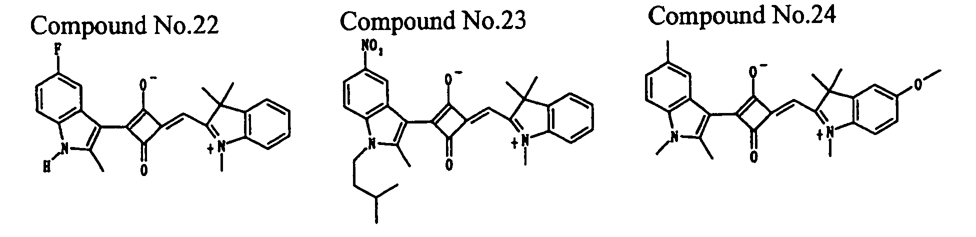

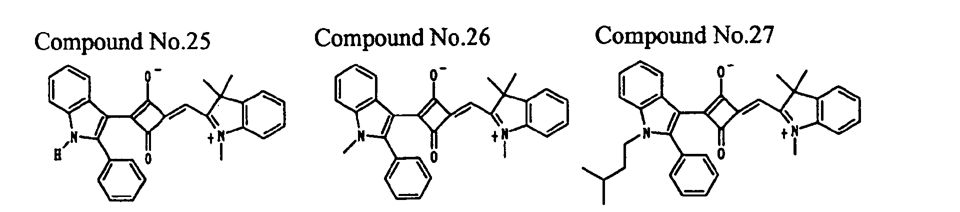

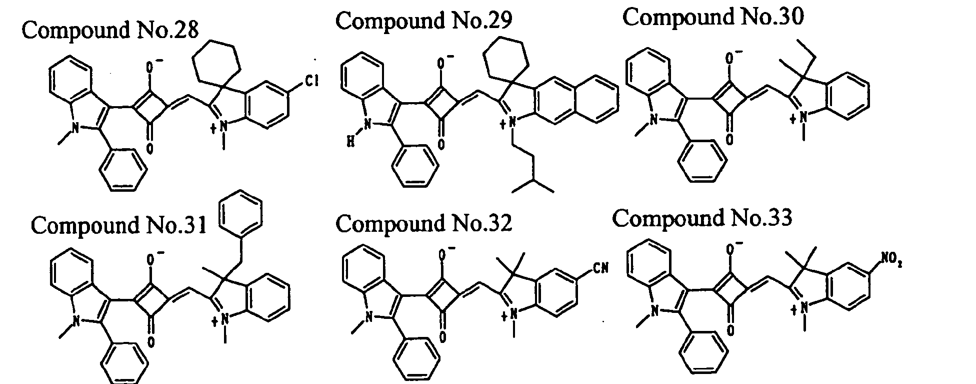

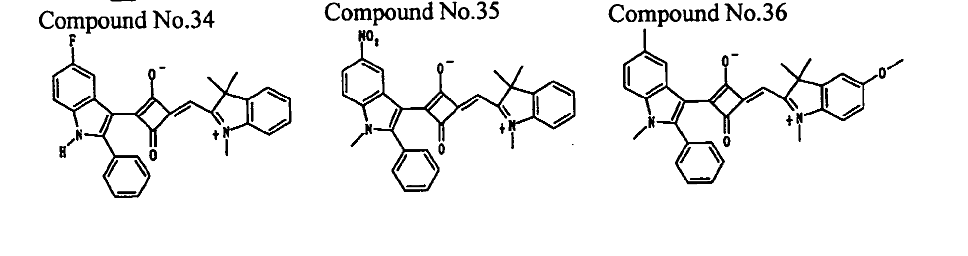

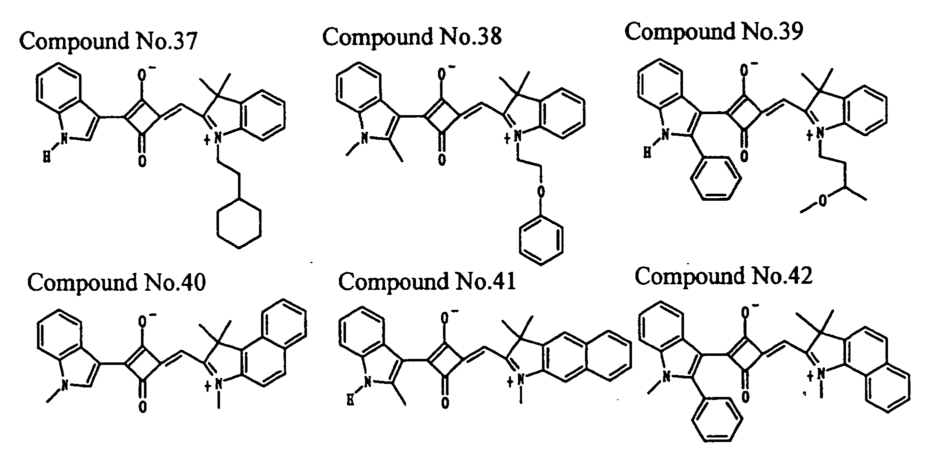

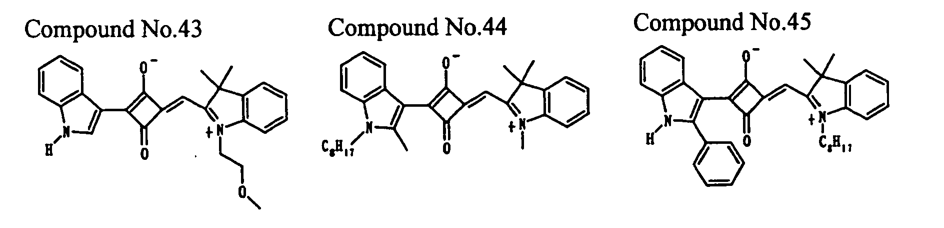

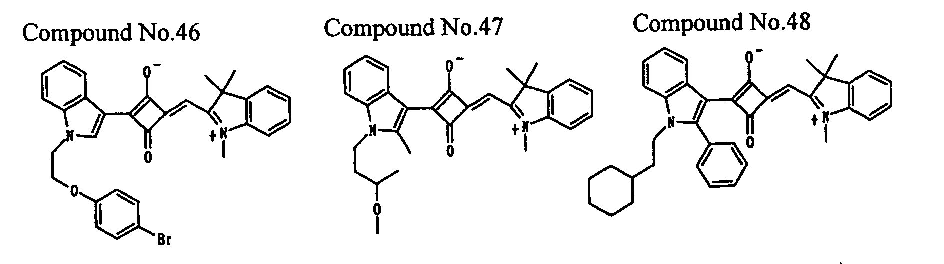

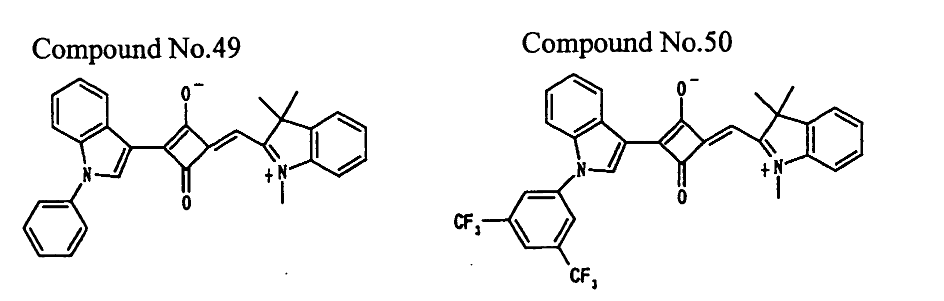

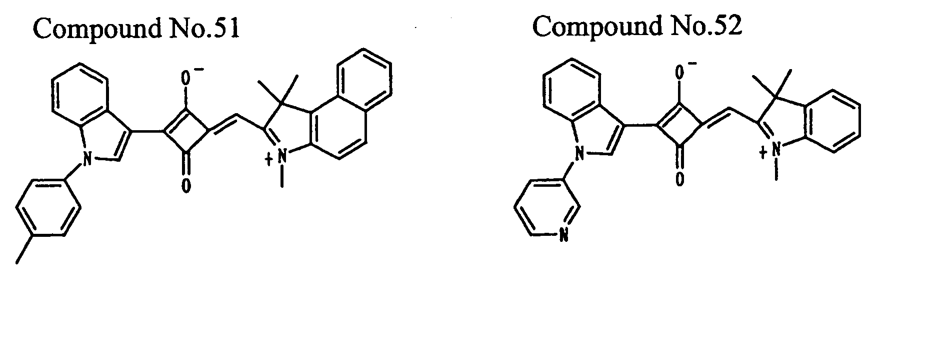

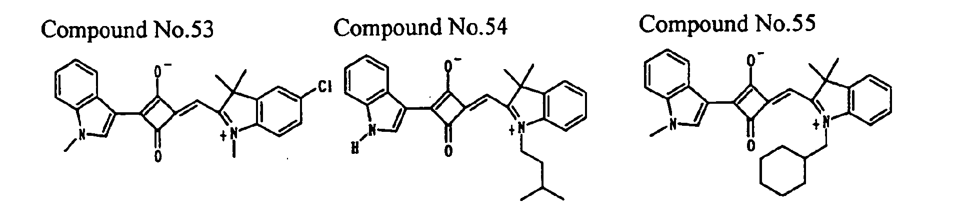

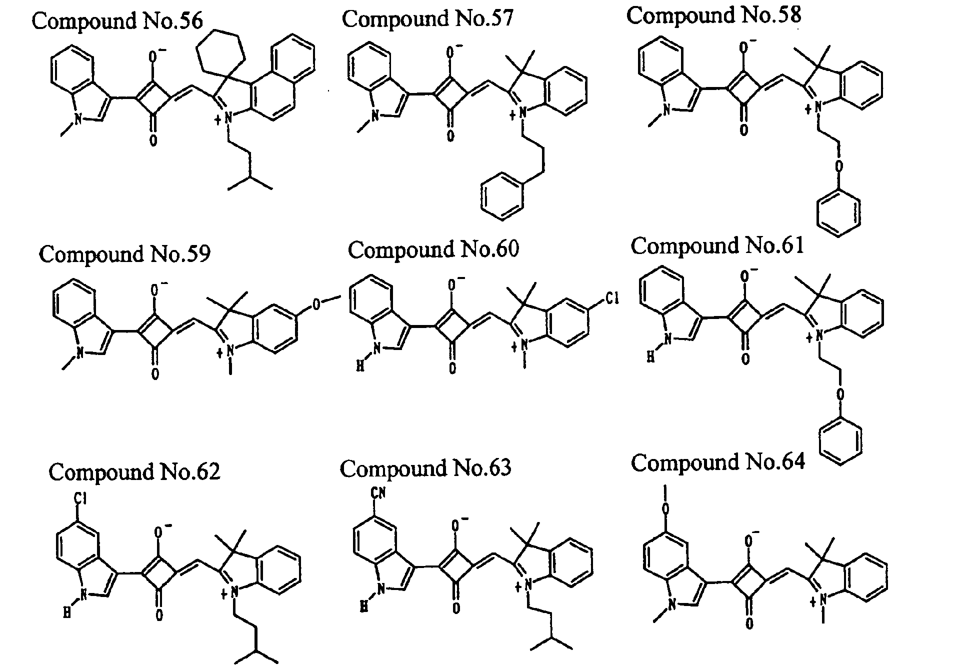

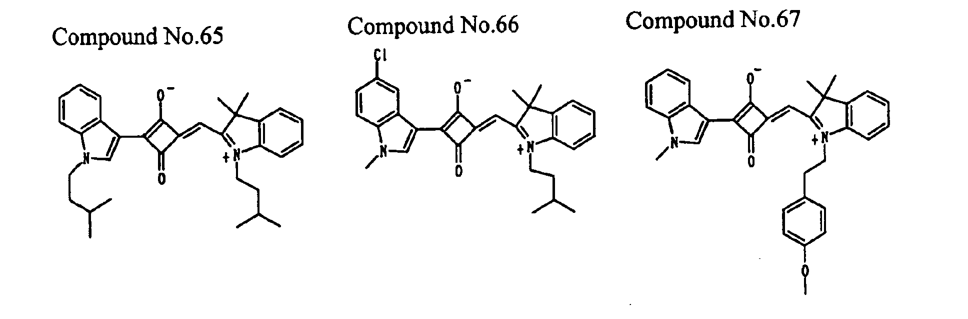

The method of preparing the cyanine compound (I) is not restricted. The cyanine compound (I) can be obtained by utilizing well-known reactions, for example, a reaction between a compound having a corresponding cyclic structure and a squaric acid derivative as in route 1 below. 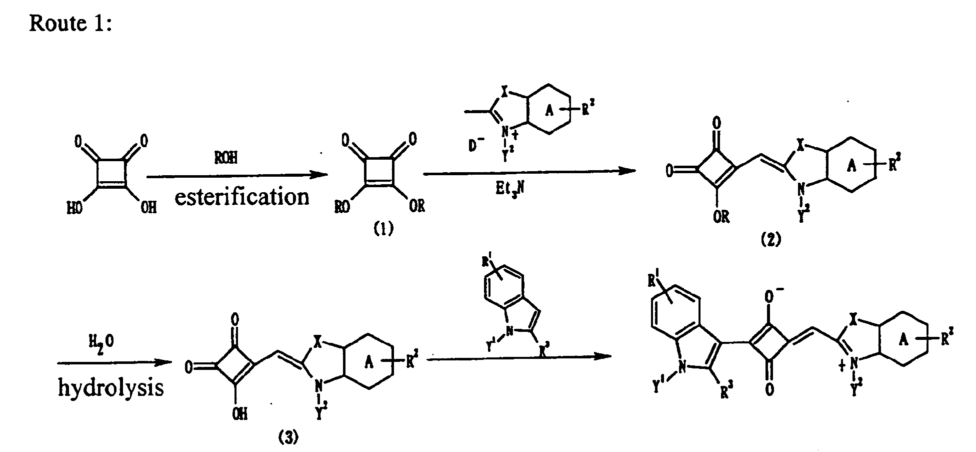 wherein ring A, R¹, R², R³, X, Y¹, and Y² are as defined above; R represents an alkyl group; and D⁻ represents a halide anion or a sulfonyloxy anion.

The halogen as D includes chlorine, bromine, and iodine, and the sulfonyloxy as D includes phenylsulfonyloxy, 4-methylsulfonyloxy, and 4-chlorosulfonyloxy.

The cyanine compound (I) is suitable as an optical element responsive to light having wavelengths of 500 to 700 nm, particularly 550 to 620 nm. The term "optical element" as used herein means an element that absorbs specific light rays to exhibit an intended function and includes a light absorber, an optical recording agent, and a photosensitizer. For instance, an optical recording agent is used in an optical recording layer of optical recording media such as DVD-Rs, and a light absorber is used in an optical filter of image displays such as LCDs, PDPs, ELDs, CRTs, fluorescent display tubes, and FEDS.

The cyanine compound (I) is characterized by high solubility in organic solvents as well as superiority in optical characteristics and stability to light and heat. These characteristics are advantageous in application to optical recording media and optical filters.

In application to optical recording media, for example, the optical recording layer of an optical disk, etc. is usually formed by coating a substrate disk with a solution of an optical recording agent in an organic solvent by spin coating or spraying. Therefore, an optical recording agent with higher organic solvent solubility is advantageous for providing wider processing latitude. Besides, a compound having high organic solvent solubility tends to have good compatibility with synthetic resins. This brings about another advantage in the manufacture of optical filters that requires uniformly dispersing or dissolving an optical element in a synthetic resin.

The optical filter according to the present invention, which contains the cyanine compound (I), will then be described.

Because the cyanine compound (I) has a narrow half bandwidth of absorption, it shows small absorption of light necessary for display. Therefore, the optical filter containing the cyanine compound (I) is especially suited for use in image display devices for the purpose of preventing malfunction with a (near) infrared remote control or improving display quality.

The optical filter of the invention is usually disposed in front of a display. It may be stuck directly to the front surface of a display or, where a front plate is provided in front of a display, it may be stuck to the front side or the back side of the front plate.

The cyanine compound (I) is used in the optical filter usually in an amount of 1 to 1000 mg/m², preferably 5 to 100 mg/m², per unit area of the optical filter. Amounts less than 1 mg/m² fail to produce sufficient effects of light absorption. Amounts exceeding 1000 mg/m² result in noticeable coloring of the filter, which can impair display quality or reduce the display brightness.

The optical filter typically comprises a transparent substrate and one or more arbitrarily layer(s) formed on the substrate, such as a primer layer, an antireflection layer, a hard coat layer and a lubricating layer. The cyanine compound (I) and other arbitrarily components, such as a color compound other than the cyanine compound (I) and various stabilizers, can be incorporated into the optical filter by (1) incorporating into the transparent substrate or an arbitrarily selected layer or layers, (2) applying to the transparent substrate or an arbitrarily selected layer or layers, (3) incorporating into an adhesive applied between adjacent layers or (4) incorporating into an independently provided light absorbing layer. The cyanine compound (I) is preferably incorporated into an adhesive applied between adjacent layers or an independently provided light absorbing layer.

The transparent substrate can be of inorganic materials such as glass and organic polymers. The organic polymers include cellulosic resins, such as diacetyl cellulose, triacetyl cellulose (TAC), propionyl cellulose, butyryl cellulose, acetylpropionyl cellulose, and nitrocellulose; polyamides; polycarbonates; polyester resins, such as polyethylene terephthalate, polyethylene naphthalate, polybutylene terephthalate, poly(1,4-cyclohexane dimethylene terephthalate), and poly(ethylene-1,2-diphenoxyethane-4,4'-dicarboxylate); polystyrenes; polyolefins, such as polyethylene, polypropylene, and polymethylpentene; acrylic resins, such as polymethyl methacrylate; polysulfones, polyether sulfones, polyether ketones, polyether imides, polyoxyethylene, and norbornene resins.

It is preferred for the transparent substrate to have a transmittance of at least 80%, particularly 86% or higher; a haze of not more than 2%, particularly 1% or less; and a refractive index of 1.45 to 1.70.

If desired, additives such as an infrared absorber, an ultraviolet absorber, and fine inorganic particles may be incorporated into the transparent substrate. The transparent substrate may be subjected to various surface treatments.

The inorganic material of the fine inorganic particles includes silicon dioxide, titanium dioxide, barium sulfate, calcium carbonate, talc, and kaolin.

The surface treatments include chemical treatments, mechanical treatments, a corona discharge treatment, a flame treatment, an ultraviolet treatment, a radiofrequency treatment, a glow discharge treatment, an active plasma treatment, a laser treatment, a mixed acid treatment, and an ozone oxidation treatment.

Where a light absorbing layer containing a light absorber is provided, a primer layer is provided between a transparent substrate and the light absorbing layer. The primer layer includes a layer of a polymer having a glass transition temperature (Tg) of -60° to 60°C, a layer with a rough surface on the light absorbing layer side thereof, and a layer of a polymer having affinity to the polymer (binder) of the light absorbing layer. Even where an independent light absorbing layer is not provided, a primer layer can be provided on a transparent substrate to improve the adhesion between the substrate and a layer provided thereon (e.g., an antireflective layer or a hard coat layer). A primer layer can also be provided in order to improve the affinity of the optical filter to an adhesive with which the optical filter is adhered to an image display device.

The thickness of the primer layer is suitably 2 nm to 20 µm, preferably 5 nm to 5 µm, still preferably 20 nm to 2 µm, particularly preferably 50 nm to 1 µm, especially preferably 80 nm to 300 nm. A primer layer containing a polymer whose Tg ranges -60° to 60°C serves to adhere the transparent substrate and an adjacent layer because of its tackiness. Polymers whose Tg is -60° to 60°C include homo- and copolymers of vinyl chloride, vinylidene chloride, vinyl acetate, butadiene, neoprene, styrene, chloroprene, acrylic esters, methacrylic esters, acrylonitrile or methyl vinyl ether. Their Tg is preferably 50°C or lower, still preferably 40°C or lower, particularly preferably 30°C or lower, especially preferably 25°C or lower. A Tg lower than 20°C is the most preferred. It is preferred for the primer layer to have an elastic modulus of 1 to 1000 MPa, particularly 5 to 800 MPa, especially 10 to 500 MPa, at 25°C.

Where a primer layer with surface roughness on its light absorber layer side is provided between a transparent substrate and a light absorbing layer, the surface roughness brings about improved adhesion between the two. Such a primer layer can easily be formed by applying a polymer latex to the transparent substrate. The polymer latex preferably has an average particle size of 0.02 to 3 µm, particularly 0.05 to 1 µm. The polymer having affinity to the polymer (binder) of the light absorbing layer includes acrylic resins, cellulose derivatives, gelatin, casein, starch, polyvinyl alcohol, soluble nylon, and polymer latices. The optical filter may have two or more primer layers. If desired, the primer layer may contain a solvent for swelling a transparent substrate, a matting agent, a surface active agent, an antistatic agent, a coating aid, a hardener, and so forth.

The antireflective layer essentially contains a low refractive layer having a lower refractive index than the transparent substrate. The refractive index of the low refractive layer is preferably 1.20 to 1.55, still preferably 1.30 to 1.50. The thickness of the low refractive layer is preferably 50 to 400 nm, still preferably 50 to 200 nm. The low refractive layer includes a layer of low-refractive, fluorine-containing polymer (see JP-A-57-34526, JP-A-3-130103, JP-A-6-115023, JP-A-8-313702, and JP-A-7-168004), a layer formed by a sol-gel process (see JP-A-5-208811, JP-A-6-299091, and JP-A-7-168003), and a layer containing fine particles (see JP-B-60-59250, JP-A-5-13021, JP-A-6-56478, JP-A-7-92306, and JP-A-9-288201). The low refractive layer containing fine particles has microvoids formed between the fine particles or inside the fine particles. The low refractive layer containing fine particles preferably has a void of 3 to 50% by volume, particularly 5 to 35% by volume.

In order to prevent reflection over a broad wavelength range, the antireflective layer preferably contains a medium to high refractive layer in addition to the low refractive layer. The refractive index of a high refractive layer is preferably 1.65 to 2.40, still preferably 1.70 to 2.20. The refractive index of a medium refractive layer is set to be the intermediate between the refractive indices of the low and the high refractive layers and is preferably 1.50 to 1.90, still preferably 1.55 to 1.70. The thickness of the medium and the high refractive layers is preferably 5 nm to 100 µm, still preferably 10 nm to 10 µm, particularly preferably 30 nm to 1 µm. The haze of the medium and the high refractive layers is preferably 5% or less, still preferably 3% or less, particularly preferably 1% or less. The medium and the high refractive layers are formed by using polymer binders having relatively high refractive indices, such as polystyrene, styrene copolymers, polycarbonates, melamine resins, phenol resins, epoxy resins, and polyurethanes obtained by the reaction between a cyclic (alicyclic or aromatic) isocyanate and a polyol. Polymers having a cyclic (aromatic, heterocyclic or alicyclic) group and polymers having a halogen atom except fluorine as a substituent also have high refractive indices. Polymers prepared from monomers having a double bond introduced therein and thereby capable of radical polymerization are also useful.

Fine inorganic particles can be dispersed in the above-recited polymer binders to have increased refractive indices. Fine inorganic particles having a refractive index of 1.80 to 2.80 are used preferably. Such fine inorganic particles are preferably prepared from metal oxides or sulfides, such as titanium oxide (including rutile, rutile/anatase mixed crystals, anatase, and amorphous oxide), tin oxide, indium oxide, zinc oxide, zirconium oxide, and zinc sulfide. Preferred of them are titanium oxide, tin oxide, and indium oxide. The fine inorganic particles may contain the metal oxide or sulfide as a major component and other elements as a minor component. The term "major component" means a component present in the particles in the highest weight proportion. Other elements that may be present in a minor proportion include Ti, Zr, Sn, Sb, Cu, Fe, Mn, Pb, Cd, As, Cr, Hg, Zn, Al, Mg, Si, P, and S. The medium or high refractive layer can also be formed by using inorganic materials that are liquid *per se* or dispersible in a solvent and are capable of forming a film, such as alkoxides of various elements, salts of organic acids, coordination compounds having a coordinating compound bonded (e.g., chelate compounds), and inorganic active polymers.

The surface of the antireflective layer may be given an antiglare function for scattering incident light into all directions thereby preventing the surrounding environment from reflecting on the antireflective layer. For example, an antireflective layer can be formed on a transparent film with fine surface roughness, or the surface of an antireflective layer can be embossed to have fine surface roughness. An antireflective layer with an antiglare function usually has a haze of 3 to 30%.

The hard coat layer has higher hardness than the transparent substrate. The hard coat layer preferably contains a crosslinked polymer. The hard coat layer can be formed by using acrylic, urethane or epoxy polymers, oligomers or monomers, such as ultraviolet curing resins. The hard coat layer can also be made of a silica-based material.

A lubricating layer may be provided on the antireflective layer (low refractive layer). A lubricating layer imparts slip properties to the surface of the low refractive layer thereby improving scratch resistance. The lubricating layer can be formed using organopolysiloxanes (e.g., silicone oils), natural waxes, petroleum waxes, higher fatty acid metal salts, or fluorine-containing lubricants or derivatives thereof. The lubricating layer preferably has a thickness of 2 to 20 nm.

Where a light absorbing layer is provided independent of the above-described layers, the light absorbing layer can be formed of the cyanine compound (I) either alone or in combination with a binder. Useful binders include naturally occurring polymers, such as gelatin, casein, starch, cellulose derivatives, and alginic acid, and synthetic polymers, such as polymethyl methacrylate, polyvinyl butyral, polyvinylpyrrolidone, polyvinyl alcohol, polyvinyl chloride, styrene-butadiene copolymers, polystyrene, polycarbonate, and polyamide.

The primer layer, antireflective layer, hard coat layer, lubricating layer, light absorbing layer, and the like can be formed by commonly employed coating methods including dip coating, air knife coating, curtain coating, roller coating, wire bar coating, gravure coating, and extrusion coating using a hopper (see U.S. Patent 2,681,294). Two or more layers can be formed by simultaneous coating. For the details of simultaneous coating techniques, reference can be made in U.S. Patents 2,761,791, 2,941,898, 3,508,947, and 3,526,528, and Harasaki Yuji, *Coating Kogaku,* Asakura Shoten, 1973, 253.

The optical recording material of the present invention, which contains the cyanine compound (I), will be described. The cyanine compound (I)-containing optical recording material of the invention is used in an optical recording layer of an optical recording medium. The optical recording medium comprises a substrate and the optical recording layer provided thereon and is capable of recording information as a pattern thermally formed with a laser beam, etc. The term "optical recording material" as used herein denotes a material used to form the optical recording layer and includes not only the cyanine compound (I) *per se* but mixtures of the cyanine compound (I) and other components, such as an organic solvent and other compounds hereinafter described.

The optical recording layer of that type of optical recording media is usually formed by wet coating processes using a solution of the cyanine compound (I) or dry coating processes such as vacuum evaporation and sputtering of the cyanine compound (I). The solution to be used in wet coating processes are prepared by dissolving the cyanine compound (I) and, if desired, other compounds in an organic solvent. Suitable organic solvents include lower alcohols, such as methanol and ethanol; ether alcohols, such as methyl cellosolve, ethyl cellosolve, butyl cellosolve, and butyl diglycol; ketones, such as acetone, methyl ethyl ketone, methyl isobutyl ketone, cyclohexanone, and diacetone alcohol; esters, such as ethyl acetate, butyl acetate, and methoxyethyl acetate; acrylic esters, such as ethyl acrylate and butyl acrylate; fluoroalcohols, such as 2,2,3,3-tetrafluoropropanol; hydrocarbons, such as benzene, toluene, and xylene; and chlorinated hydrocarbons, such as methylene dichloride, dichloroethane, and chloroform. The solution can be applied by spin coating, spraying, dipping, and like methods.

The thickness of the optical recording layer is usually 0.001 to 10 µm, preferably 0.01 to 5 µm.

The content of the cyanine compound (I) in the optical recording material is preferably 50 to 100% by weight on a solid basis.

If desired, the optical recording material can contain compounds commonly employed in an optical recording layer, such as cyanine compounds other than those of formula (I), azo compounds, and phthalocyanine compounds. The optical recording material can further contain resins, such as polyethylene, polyester, polystyrene, and polycarbonate, surface active agents, antistatic agents, lubricants, flame retardants, radical scavengers (e.g., hindered amines), pit formation accelerators (e.g., ferrocene derivatives), dispersants, antioxidants, crosslinking agents, light resistance imparting agents, and so forth. The optical recording material can furthermore contain an aromatic nitroso compound, an aluminum compound, an iminium compound, a bisiminium compound, a transition metal chelate compound, and the like as a quencher for singlet oxygen, etc. For the same purpose, a quencher anion may be used. These various compounds other than cyanine compound (I) can be present in the optical recording material in a total amount of up to 50% by weight on a solid basis.

A reflective layer of gold, silver, aluminum, copper, etc. may be formed on the optical recording layer by vacuum evaporation or sputtering. A protective layer of an acrylic resin, an ultraviolet cured resin, etc. may be provided on the optical recording layer.

The present invention will now be illustrated in greater detail with reference to Preparation Examples, Evaluation Examples, Examples, and Comparative Examples, but it should be understood that the invention is not construed as being limited thereto.

### PREPARATION EXAMPLE 1

### Synthesis of Compound No. 1:

In a reaction flask purged with nitrogen were put 0.088 mol of squaric acid, 60 g of 1-butanol, and 30 g of toluene, and the mixture was refluxed at 110°C while removing water produced. After a theoretical amount of water was removed, 20 ml of the solvent was removed by evaporation while blowing nitrogen gas. After confirming the absence of squaric acid in the reaction system, the solvent was completely removed to give dibutyl squarate (compound (1) in route 1) in a yield of 100%.

In a reaction flask purged with nitrogen were charged 0.053 mol of an iodide of a 2-methylindolium derivative, 0.079 mol of triethylamine, and 86 g of ethanol and mixed uniformly by stirring at room temperature. To the mixture was added dropwise at room temperature 0.053 mol of the above prepared dibutyl squarate, followed by stirring at room temperature for an additional 5 hour period. The reaction mixture was allowed to stand still, and the precipitated crystals were washed with methanol to give an intermediate product (a) (compound (2) in route 1) in a yield of 86%.

In a reaction flask purged with nitrogen were put 0.039 mol of the intermediate product (a), 104 g of acetic acid, and 52 g of water, and the mixture was refluxed at 100°C for 5 hours. The reaction mixture was concentrated to dryness. The resulting solid was washed with ethyl acetate to afford an intermediate product (b) (compound (3) in route 1) in a yield of 83%.

In a reaction flask purged with nitrogen were put 0.012 mol of the intermediate product (b), 0.012 mol of an indole derivative, 27 g of 1-butanol, and 13.5 g of toluene. The mixture was allowed to react at 80 to 85°C while blowing nitrogen gas and removing the solvent by evaporation until the peak assigned to the intermediate product (b) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 1 as titled in a yield of 85%.

The product as obtained was analyzed to give the following results and identified to be compound No. 1.

### Results of Analyses:

¹H-NMR (DMSO) (chemical shift (ppm), multiplicity, and number of protons of the peak top): (1.83, s, 6), (3.86, s, 3), (4.00, s, 3), (6.11, s, 1), (7.31-7.43, m, 3), (7.51-7.55, t, 1), (7.61-7.66, t, 2), (7.72-7.74, d, 1), (8.45, s, 1), (8.70-8.72, d, 2)
IR Absorption (particularly large absorptions between 4000 cm⁻¹ and 1000 cm⁻¹): 1604, 1499,1458,1414,1297,1228,1090,1061
Elemental Analysis:
Calcd. (%): C 78.5; H 5.80; N 7.32
Found (%): C 78.3; H 5.78; N 7.30
UV Absorption (chloroform): λₘₐₓ: 587 nm; ε: 1.76x10⁵
Decomposition Temperature in TG-DTA (100 ml/min nitrogen stream; temperature rise: 10°C/min): 281°C

### PREPARATION EXAMPLE 2

### Synthesis of Compound No. 2:

In a reaction flask purged with nitrogen were put 0.006 mol of the intermediate product (b) prepared in the same manner as in Preparation Example 1, 0.006 mol of an indole derivative, 13 g of 1-butanol, and 6.5 g of toluene. The mixture was allowed to react at 80 to 85°C while blowing nitrogen gas and removing the solvent by evaporation until the peak assigned to the intermediate product (b) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 2 as titled in a yield of 30%.

The results of analyses on the product as obtained for identification are shown below.
¹H-NMR (DMSO) (chemical shift (ppm), multiplicity, number of protons): (1.86, s, 6), (3.88, s, 3), (6.14, s, 1), (7.28-7.35, m, 2), (7.44-7.48, t, 1), (7.56-7.58, d, 2), (7.67-7.69, d, 1), (7.75-7.77, d, 1), (8.42, s, 1), (8.71-8.73, d, 1), (12.36, s, 1)
IR Absorption (particularly large absorption between 4000 cm⁻¹ and 1000 cm⁻¹): 1597, 1499,1476,1456,1436,1417,1315,1196,1179,1116,1063
Elemental Analysis:
Calcd. (%): C 78.2; H 5.47; N 7.60
Found (%): C 78.2; H 5.44; N 7.51
UV Absorption (chloroform): λₘₐₓ: 578 nm; ε: 1.41x10⁵
Decomposition Temperature in TG-DTA (100 ml/min nitrogen stream; temperature rise: 10°C/min): 286°C

### PREPARATION EXAMPLE 3

### Synthesis of Compound No. 4:

Into a reaction flask purged with nitrogen were charged 0.044 mol of a toluenesulfonate of a 2-methylindolium derivative, 0.066 mol of triethylamine, and 74 g of ethanol and mixed uniformly by stirring at room temperature. To the mixture was added dropwise at room temperature 0.044 mol of dibutyl squarate prepared in the same manner as in Preparation Example 1, followed by stirring for additional 5 hours at room temperature. The reaction system was allowed to stand, and the precipitated crystals were washed with methanol to give an intermediate product (c) (compound (2) in route 1) in a yield of 79%.

In a reaction flask purged with nitrogen were put 0.034 mol of the resulting intermediate product (c), 120 g of acetic acid, and 60 g of water, and the mixture was refluxed at 100°C for 5 hours. The reaction mixture was concentrated to dryness, and the resulting solid was washed with ethyl acetate to furnish an intermediate compound (d) (compound (3) in route 1) in a yield of 40%.

In a reaction flask purged with nitrogen were put 0.0056 mol of the intermediate product (d), 0.0056 mol of an indole derivative, 26 g of 1-butanol, and 13.0 g of toluene. The mixture was allowed to react at 80 to 85°C while blowing nitrogen gas and removing the solvent by evaporation until the peak assigned to the intermediate product (d) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 4 in a yield of 30%.

The product as obtained was analyzed to give the following results and identified to be compound No. 4.

### Results of Analyses:

¹H-NMR (DMSO) (chemical shift (ppm), multiplicity, number of protons): (1.85-2.11, m, 8), (2.4-2.75; m, 2), (3.98, s, 3), (3.27, s, 3), (6.32, s, 1), (7.26-7.30, t, 1), (7.34-7.37, t, 1), (7.57-7.60, d, 1), (7.69-7.73, t, 1), (7.83-7.86, t, 1), (7.97-8.00, d, 1), (8.24-8.30, m, 3), (8.43-8.45, d, 1), (8.67-8.69, d, 1)
IR Absorption (particularly large absorption between 4000 cm⁻¹ and 1000 cm⁻¹): 3450, 1601, 1490, 1469, 1440, 1349, 1317, 1231, 1100, 1093, 1078
Elemental Analysis:
Calcd. (%): C 81.3; H 5.97; N 5.93
Found (%): C 81.1; H 5.95; N 5.89
UV Absorption (chloroform): λₘₐₓ: 607 nm; ε: 1.01x10⁵
Decomposition Temperature in TG-DTA (100 ml/min nitrogen stream; temperature rise: 10°C/min): 259°C

### PREPARATION EXAMPLE 4

### Synthesis of Compound No. 5:

In a reaction flask purged with nitrogen were charged 0.030 mol of an iodide of a 2-methylindolium derivative, 0.045 mol of triethylamine, and 45 g of ethanol and mixed uniformly by stirring at room temperature. To the mixture was added dropwise at room temperature 0.030 mol of dibutyl squarate prepared in the same manner as in Preparation Example 1, followed by stirring at room temperature for an additional 5 hour period. The reaction mixture was allowed to stand, and the precipitated crystals were washed with methanol to give an intermediate product (e) (compound (2) in route 1) in a yield of 64%.

In a reaction flask purged with nitrogen were put 0.018 mol of the intermediate product (e), 60 g of acetic acid, and 30 g of water, and the mixture was refluxed at 100°C for 5 hours. The reaction mixture was concentrated to dryness. The resulting solid was washed with ethyl acetate to afford an intermediate product (f) (compound (3) in route 1) in a yield of 70%.

In a reaction flask purged with nitrogen were put 0.006 mol of the intermediate product (f), 0.006 mol of an indole derivative, 28 g of 1-butanol, and 14 g of toluene. The mixture was allowed to react at 80 to 85°C while blowing nitrogen gas and removing the solvent by evaporation until the peak assigned to the intermediate product (f) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 5 in a yield of 61%.

The results of identification analyses on the product are shown below.
¹H-NMR (DMSO) (chemical shift (ppm), multiplicity, number of protons): (1.09-1.11, d, 6), (1.69-1.75, m, 2), (1.82-1.90, m+s, 7),(4.03, s, 3), (4.34-4.38, t, 2), (6.15, s, 1), (7.33-7.45, m, 3), (7.53-7.57, t, 1), (7.62-7.66, m, 2), (7.75-7.77, d, 1), (8.47, s, 1), (8.72-8.73, d, 1)
IR Absorption (particularly large absorption between 4000 cm⁻¹ and 1000 cm⁻¹): 3434, 1600,1495,1452,1415,1306,1287,1230,1196,1092,1063
Elemental Analysis:
Calcd. (%): C 79.4; H 6.89; N 6.39
Found (%): C 79.0; H 6.87; N 6.35
UV Absorption (chloroform): λₘₐₓ: 587 nm; ε: 1.88x10⁵
Decomposition Temperature in TG-DTA (100 ml/min nitrogen stream; temperature rise: 10°C/min): 271°C

### PREPARATION EXAMPLE 5

### Synthesis of Compound No. 14:

In a reaction flask purged with nitrogen were put 0.01 mol of the intermediate product (b) prepared in the same manner as in Preparation Example 1, 0.01 mol of an indole derivative, 28 g of 1-butanol, and 14 g of toluene. The mixture was allowed to react at 80 to 85°C while blowing nitrogen gas and removing the solvent by evaporation until the peak assigned to the intermediate product (b) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 14 in a yield of 92%.

The results of identification analyses on the product as obtained are shown below.
¹H-NMR (DMSO) (chemical shift (ppm), multiplicity, number of protons): (1.84, s, 6), (3.23, s, 3), (3.84, s, 3), (3.85, s, 3), (6.08, s, 1), (7.24-7.33, m, 2), (7.39-7.42, t, 1), (7.52-7.58, m, 2), (7.62-7.64, d, 1), (7.71-7.73, d, 1), (9.01-9.03, d, 1)
IR Absorption (particularly large absorption between 4000 cm⁻¹ and 1000 cm⁻¹): 1600, 1480, 1453, 1428, 1392, 1296, 1210, 1087, 1068
Elemental Analysis:
Calcd. (%): C 78.8; H 6.10; N 7.07
Found (%): C 78.5; H 6.05; N 7.07
UV Absorption (chloroform): λₘₐₓ: 602 nm; ε: 1.79x10⁵
Decomposition Temperature in TG-DTA (100 ml/min nitrogen stream; temperature rise: 10°C/min): 287°C

### PREPARATION EXAMPLE 6

### Synthesis of Compound No. 22:

In a reaction flask purged with nitrogen were put 0.006 mol of the intermediate product (b) prepared in the same manner as in Preparation Example 1, 0.006 mol of an indole derivative, 13 g of 1-butanol, and 6.5 g of toluene. The mixture was allowed to react at 80 to 85°C while blowing nitrogen gas and removing the solvent by evaporation until the peak assigned to the intermediate product (b) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 22 in a yield of 88%.

The results of identification analyses on the product as obtained are shown below.
¹H-NMR (DMSO) (chemical shift (ppm), multiplicity, number of protons): (1.85, s, 6), (3.13, s, 3), (3.87, s, 3), (6.10, s, 1), (7.04-7.9, m, 1), (7.38-7.45, m, 2), (7.53-7.57, t, 1), (7.65-7.67, d, 1), (7.73-7.75, d, 1), (8.75-8.78, d, 1), (12.3, s, 1)
IR Absorption (particularly large absorption between 4000 cm⁻¹ and 1000 cm⁻¹): 1598, 1567, 1452, 1425, 1374, 1350, 1291, 1241, 1201, 1156, 1097, 1072, 1000
Elemental Analysis:
Calcd. (%): C 75.0; H 5.29; N 7.00
Found (%): C 74.7; H 5.32; N 7.02
UV Absorption (chloroform): λₘₐₓ: 590 nm; ε: 1.49x10⁵
Decomposition Temperature in TG-DTA (100 ml/min nitrogen stream; temperature rise: 10°C/min): 295°C

### PREPARATION EXAMPLE 7

### Synthesis of Compound No. 26:

In a reaction flask purged with nitrogen were put 0.0038 mol of the intermediate product (b) prepared in the same manner as in Preparation Example 1, 0.0038 mol of an indole derivative, 34 g of 1-butanol, and 17 g of toluene. The mixture was allowed to react at 80 to 85°C while blowing nitrogen gas and removing the solvent by evaporation until the peak assigned to the intermediate product (b) disappeared in HPLC analysis. The reaction mixture was cooled to room temperature, and ethyl acetate was added thereto. The crystals thus precipitated were collected by filtration, washed with ethyl acetate, and dried in vacuo at 160°C to give compound No. 26 in a yield of 60%.

The results of identification analyses on the product as obtained are shown below.
¹H-NMR (DMSO) (chemical shift (ppm), multiplicity, number of protons): (1.76, s, 6), (3.74, s, 3), (3.82, s, 3), (6.04, s, 1), (7.30-7.34, t, 1), (7.37-7.43, m, 2), (7.50-7.54, m, 6), (7.63-7.64, d, 1), (7.65-7.66, d, 1), (7.70-7.72, d, 1), (8.86-8.88, d, 1)
IR Absorption (particularly large absorption between 4000 cm⁻¹ and 1000 cm⁻¹): 1604, 1573,1475,1456,1436,1380,1348,1294,1243,1218,1195,1091,1066,1018
Elemental Analysis:
Calcd. (%): C 81.2; H 5.72; N 6.11
Found (%): C 81.5; H 5.69; N 6.13
UV Absorption (chloroform): λₘₐₓ: 606 nm; ε: 1.45x10⁵
Decomposition Temperature in TG-DTA (100 ml/min nitrogen stream; temperature rise: 10°C/min): 273°C

### PREPARATION EXAMPLE 8

### Synthesis of Compound No. 65:

Into a reaction flask purged with nitrogen were charged 0.090 mol of an iodide of a 2-isoamylindolium derivative, 0.135 mol of triethylamine, and 135 g of ethanol and mixed uniformly by stirring at room temperature. To the mixture was added dropwise at room temperature 0.090 mol of dibutyl squarate prepared in the same manner as in Preparation Example 1, followed by stirring for additional 5 hours at room temperature. The reaction system was allowed to stand, and the precipitated crystals were washed with methanol to give an intermediate product (g) (compound (2) in route 1) in a yield of 60%.

In a reaction flask purged with nitrogen were put 0.050 mol of the resulting intermediate product (g), 180 g of acetic acid, and 90 g of water, and the mixture was refluxed at 100°C for 5 hours. The reaction mixture was concentrated to dryness, and the resulting solid was washed with ethyl acetate to furnish an intermediate compound (h) (compound (3) in route 1) in a yield of 70%.

In a reaction flask purged with nitrogen were put 0.012 mol of the intermediate product (h), 0.013 mol of an indole derivative, 17 g of isoamyl alcohol, and 17 g of toluene. The mixture was allowed to react at 80 to 85°C while blowing nitrogen gas and removing the solvent by evaporation until the peak assigned to the intermediate product (h) disappeared in HPLC analysis. The reaction mixture was cooled to room temperature, and ethyl acetate was added thereto. The crystals thus precipitated were collected by filtration and purified by silica gel column chromatography to give compound No. 65 in a yield of 47%.

The results of identification analyses on the product are shown below.
¹H-NMR (CDCl₃) (chemical shift (ppm), multiplicity, number of protons): (0.95-0.97, d, 6), (1.04-1.06, d, 6), (1.59-1.76, m+m, 4), (1.78-1.80, m+m, 2), (1.83, s, 6), (4.01-4.12, t, 2), (4.15-4,19, t, 2), (6.14, s, 1), (7.08-7.10, d, 1), (7.20-7.39, m, 5), (7.41-7.43, d, 1), (8.38, s, 1), (8.80-8.82, m, 1)
IR Absorption (particularly large absorption between 4000 cm⁻¹ and 1000 cm⁻¹): 2958, 2919, 2869, 1601, 1576, 1493, 1457, 1393, 1352, 1308, 1288, 1237, 1190, 1107, 1080, 1063
Elemental Analysis:
Calcd. (%): C 81.1; H 7.76; N 5.66
Found (%): C 82.1; H 7.80; N 5.80
UV Absorption (chloroform): λₘₐₓ: 590 nm; ε: 1.90x10⁵
Melting Temperature in TG-DTA (100 ml/min nitrogen stream; temperature rise: 10°C/min): 221°C

### PREPARATION EXAMPLE 9

### Synthesis of Compound No. 66:

In a reaction flask purged with nitrogen were put 0.010 mol of the intermediate product (h) prepared in the same manner as in Preparation Example 8, 0.011 mol of an indole derivative, 14 g of 1-butanol, and 14 g of toluene. The mixture was allowed to react at 80 to 85°C while blowing nitrogen gas and removing the solvent by evaporation until the peak assigned to the intermediate product (h) disappeared in HPLC analysis. The reaction mixture was concentrated, and the residue was purified by silica gel column chromatography. The resulting crude product was recrystallized from ethyl acetate to give compound No. 66 in a yield of 53%.

The results of identification analyses on the product as obtained are shown below.
¹H-NMR (DMSO) (chemical shift (ppm), multiplicity, number of protons): (0.99-1.01, d, 6), (1.60-1.16, q, 2), (1.75-1.80, s+m, 7), (3.92, s, 3), (4.26-4.30, t, 2), (6.08, s, 1), (7.27-7.32, d, 1), (7.32-7.36, t, 1), (7.41-7.46, t, 1), (7.51-7.59, d+d, 2), (7.66-7.68, d, 1), (8.35, s, 1), (8.68, s, 1)
IR Absorption (particularly large absorption between 4000 cm⁻¹ and 1000 cm⁻¹): 2958, 2924, 2868, 1611, 1572, 1498, 1458, 1352, 1316, 1267, 1238, 1197, 1101, 1065
Elemental Analysis:
Calcd. (%): C 73.6; H 6.18; N 5.92
Found (%): C 74.0; H 6.24; N 6.01
UV Absorption (chloroform): λₘₐₓ: 584 nm; ε: 1.78x10⁵
Melting Temperature in TG-DTA (100 ml/min nitrogen stream; temperature rise: 10°C/min): 252°C

### EVALUATION EXAMPLE 1

### Evaluation of Solubility:

The compounds obtained in Preparation Examples 1 to 5 and 7 and comparative compounds 1 to 3 shown below were evaluated for solubility in methyl ethyl ketone (MEK). The test compound was added to MEK at 20 °C in an amount corresponding to a concentration varying from 0.1 to 0.3% by weight by 0.05% by weight to see whether it dissolved or not. The results obtained are shown in Table 1. 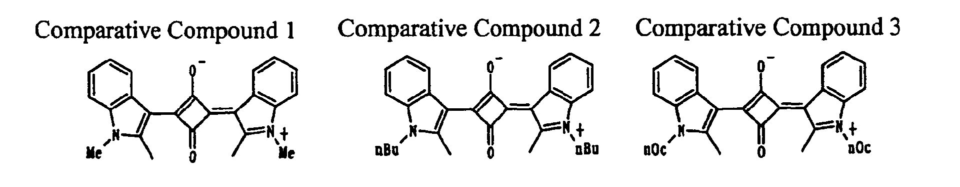 wherein Me is a methyl group; nBu represents an n-butyl group; and nOc represents an n-octyl group.

**TABLE 1**

| Prepn Example | Test Compound | Solubility |
|---|---|---|
| 1 | Compound No. 1 | soluble at 0.2%, insoluble at 0.25% |
| 2 | Compound No. 2 | soluble at 0.3% |
| 3 | Compound No. 4 | soluble at 0.15%, insoluble at 0.2% |
| 4 | Compound No. 5 | soluble at 0.3% |
| 5 | Compound No. 14 | soluble at 0.3% |
| 7 | Compound No. 26 | soluble at 0.3% |
| - | Comp. Compound 1 | insoluble at 0.1% |
| - | Comp. Compound 2 | insoluble at 0.1% |
| - | Comp. Compound 3 | soluble at 0.1%, insoluble at 0.15% |

The results in Table 1 prove that the cyanine compounds according to the present invention are superior to analogous compounds in solubility in an organic solvent.

### EVALUATION EXAMPLE 2

### Evaluation of Heat Resistance:

Ten milligrams of compound No. 1 or comparative compound 3, 150 mg of a 10 wt% chloroform solution of polycarbonate (KEL-GEF available from Takiron Co., Ltd.), and 3000 mg of chloroform were mixed up. The resulting solution was applied to a 20 mm x 20 mm glass plate by spin coating at 1500 rpm for 60 seconds to prepare a specimen. The UV absorption spectrum of the specimen was measured.

The specimen was put in a hot air circulating drier at 120°C for 41 hours. The UV absorption spectrum of the thus heated specimen was again measured to obtain an absorption retention (%) (absorption at λₘₐₓ after heating/absorption at λₘₐₓ before heating x 100). The absorption retention of the specimen of compound No. 1 was 83%, whereas that of the specimen containing comparative compound 3 was 50%, revealing the superior heat resistance of the cyanine compound (I).

### EVALUATION EXAMPLE 3

### Evaluation of Light Resistance:

The test compound shown in Table 2 below was dissolved in a 1:1 (by volume) mixed solvent of MEEK and 2,2,3,3-tetrafluoropropan-1-ol to prepare a 1 wt% solution. The solution was applied to a 20 mm x 20 mm polycarbonate plate by spin coating at 2000 rpm for 60 seconds to prepare a specimen. The UV absorption spectrum of the specimen was determined. The specimen was then irradiated with light of 55000 lux, and the exposure time required for the absorption retention (as defined in Evaluation Example 2) to decrease to 50% was measured. The results obtained are shown in Table 2.

**TABLE 2**

| Test Compound | Absorption Retention Halving Time (hr) |
|---|---|
| Compound No. 1 | 58 |
| Compound No. 3 | 26 |
| Compound No.5 | 38 |
| Comp. Compound 4* | 1.2 |

| | |
|---|---|
| * Comparative Compound 4: | |

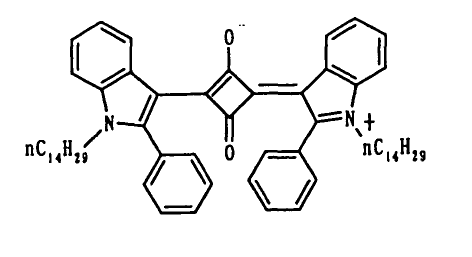

As can be seen from Table 2, the cyanine compounds of the present invention show slower reduction of absorbance than the comparative compound and are superior in light resistance.

### EXAMPLE 1

### Formulation:

| | |
|---|---|
| Iupilon S-3000 (polycarbonate resin available from Mitsubishi Gas Chemical Company, Inc.) | 100 g |
| Compound No. 1 | 0.01 g |

The components of the above formulation were melt kneaded in Labo Plastomill at 260°C for 5 minutes, and the blend was extruded through nozzles of 6 mm in diameter, cooled in water, and pelletized to obtain dye-containing pellets. The pellets were molded at 250°C in an electric press into a 0.25 mm thin plate. The thin plate had a λₘₐₓ of 588 nm with a half bandwidth of 29 nm as measured with a spectrophotometer U-3010 supplied from Hitachi, Ltd., proving suitable as an optical filter.

### EXAMPLE 2

### Formulation:

| | |
|---|---|
| ADEKA Optomer KRX-571-65 (UV curing resin (resin content: 80 wt%) available from Asahi Denka Co., Ltd.) | 100 g |
| Compound No. 3 | 0.5 g |
| Methyl ethyl ketone | 60 g |

A UV curing varnish was prepared from the above formulation. The varnish was applied to a 188 µm thick polyethylene terephthalate (PET) film having been surface treated for adhesion improvement with a bar coater #9 and dried at 80°C for 30 seconds. The coating film was irradiated with ultraviolet light from a high pressure mercury lamp equipped with an IR cut filter to obtain a cured film having a thickness of about 5 µm. The cured film had a λₘₐₓ of 590 nm with a half bandwidth of 29 nm as measured with a spectrophotometer U-3010, proving suitable as an optical filter.

### EXAMPLE 3

### Formulation:

| | |
|---|---|
| ADEKA ARKLS R-103 (acrylic resin binder (resin content: 50 wt%) available from Asahi Denka Co., Ltd.) | 100 g |
| Test compound (see Table 3) | 0.1 g |

A binder composition having the above formulation was applied to a 188 µm thick PET film having been surface treated for adhesion improvement with a bar coater #9 and dried at 80°C for 30 seconds. The PET film was hot bonded to a 0.9 mm thick alkali glass plate to prepare a PET-protected glass plate having a light absorbing dye-containing binder layer between the glass substrate and the PET film. The λₘₐₓ and the half bandwidth of the resulting PET-protected glass plate are shown in Table 3. The PET-protected glass plates prepared were proved from these results to be suitable as an optical filter.

**TABLE 3**

| Run No. | Test Compound | λₘₐₓ (nm) | Half Bandwidth (nm) |
|---|---|---|---|
| 1 | Compound No. 5 | 587 | 29 |
| 2 | Compound No. 14 | 602 | 30 |
| 3 | Compound No. 22 | 590 | 33 |
| 4 | Compound No. 23 | 597 | 36 |
| 5 | Compound No. 26 | 606 | 34 |

As is apparent from the results in Examples 1 to 3, it has been confirmed that the optical filter containing the cyanine compound (I) of the present invention exhibits a sharp absorption peak with a half bandwidth of 50 nm or narrower in a specific wavelength range (550 to 620 nm).

### EXAMPLE 4

Specimens were prepared in the same manner as in Evaluation Example 3. The UV transmission spectrum and the UV reflection spectrum (incidence angle: 5°) of the specimens were measured. The results obtained are shown in Table 4 below. In Table 4, the reflected light intensity at 650 nm is expressed relatively to that at λₘₐₓ.

**TABLE 4**

| Run No. | Test Compound | Transmitted Light λₘₐₓ (nm) | Reflected Light λₘₐₓ (nm) | Reflected Light Intensity at 650 nm (%) |
|---|---|---|---|---|
| 1 | Compound No. 1 | 610 | 659 | 95.7 |
| 2 | Compound No. 3 | 608 | 651 | 100 |
| 3 | Compound No. 5 | 611 | 656 | 95.9 |

In reading optical recording media typified by optical disks, a laser beam is reflected on an optical recording medium, and the record is detected as a difference in light quantity at the laser wavelength. Accordingly, a dye compound showing a high absorption intensity near the laser beam wavelength is preferred as an optical recording material. The results in Table 4 reveal that the cyanine compound of the present invention is a suitable optical recording material for use in optical recording media using a 650 nm recording wavelength, such as DVD-Rs.

The present invention provides a novel cyanine compound (I) useful as an optical element and excellent in heat resistance and light resistance. The optical filter comprising the cyanine compound (I) is suited for use in image display devices. The optical recording material containing the cyanine compound (I) is suitable for the formation of an optical recording layer of an optical recording medium.

## Claims

1. A cyanine compound represented by formula (I): 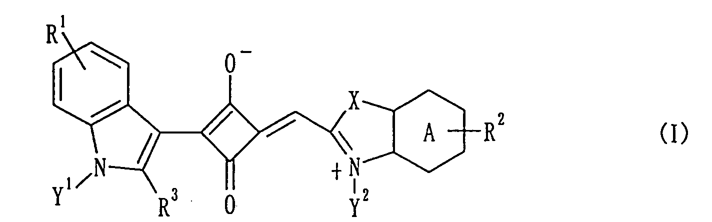 wherein ring A represents a benzene ring or a naphthalene ring; R¹ and R² each independently represent a hydrogen atom, a halogen atom, a nitro group, a cyano group, an alkyl group having 1 to 8 carbon atoms, an alkoxy group having 1 to 8 carbon atoms or an aryl-containing group having 6 to 30 carbon atoms; R³ represents a hydrogen atom, an alkyl group having 1 to 8 carbon atoms or an aryl-containing group having 6 to 30 carbon atoms; X represents an oxygen atom, a sulfur atom, a selenium atom, -CR⁴R⁵-, -NH- or -NY'-; Y¹ and Y² each independently represent a hydrogen atom or an organic group having 1 to 30 carbon atoms; R⁴ and R⁵ each independently represent an alkyl group having 1 to 4 carbon atoms or a benzyl group, or R⁴ and R⁵ are taken together to form a cycloalkane-1,1-diyl group having 3 to 6 carbon atoms; and Y' represents an organic group having 1 to 30 carbon atoms.

2. The cyanine compound according to claim 1, wherein X is -CR⁴R⁵-.

3. An optical filter containing the cyanine compound according to claim 1 or 2.

4. The optical filter according to claim 3, which is used for an image display device.

5. An optical recording material for use in an optical recording medium having a substrate and an optical recording layer formed on the substrate, the optical recording material containing the cyanine compound according to claim 1 or 2 and being used in the optical recording layer.
